# EUROPEAN PATENT APPLICATION

(11) **EP 4 360 461 A1**
(43) Date of publication of application: **01.05.2024**
(21) Application number: 22204570.0
(22) Date of filing: 28.10.2022
(51) Int. Cl.: A23B 4/12, C12N 1/20

(54) **ANTIMICROBIAL FERMENT AS FOOD INGREDIENT**

(71) Applicant: Nederlandse Organisatie voor toegepast-natuurwetenschappelijk Onderzoek TNO, 2595 DA 's-Gravenhage (NL)
(72) Inventor: Van der Vossen, Josephus Mauritius Bernardus Maria, 2595 DA 's-Gravenhage (NL); De Boer, Paulo, 2595 DA 's-Gravenhage (NL); Kieboom, Jasper, 2595 DA 's-Gravenhage (NL)
(74) Representative: V.O.

(57) **Abstract**

The invention is directed to a method for providing a foodstuff preservative. More specifically, to a foodstuff preservative that inhibits growth of *Escherichia, Salmonella* and/or *Listeria.* The invention is further directed to a foodstuff preservative obtainable by the method. The invention is further related to a method for screening antimicrobial effects of a ferment, a method for providing a preserved foodstuff and the use of a foodstuff preservative.

## Description

The invention is directed to a method for providing a foodstuff preservative. More specifically, to a foodstuff preservative that inhibits growth of *Escherichia, Salmonella* and/or *Listeria.* The invention is further directed to a foodstuff preservative obtainable by the method. The invention is further related to a method for screening antimicrobial effects of a ferment, a method for providing a preserved foodstuff and the use of a foodstuff preservative.

The quality and safety of foods relies to a large extent on microbiological stability and the absence of food pathogenic organisms. Typical food pathogenic organisms include bacteria such as *Escherichia, Salmonella* and *Listeria.* Preservatives are therefore often added to foods to increase their shelf life. The increased shelf life provides for more time for the logistics from e.g. factory to customer, but also for the customer to keep the food before consumption. However, the preservatives that are currently used may be perceived as undesired additives by consumers.

As an alternative for conventional preservatives, solutions such as ferments have been proposed. An example where fermentation is used for a method for preserving foodstuff is provided in WO2022/003120. This publication is directed to a composition for control of microbial development of an edible substance. The composition comprises a bacteriocin and an organic acid produced from fermentation of a bacteriocin producing bacteria.

Further examples are provided in KR101776511, KR2018/0021396 and KR101806720.

It is an object of the present inventors to provide a food preservative that inhibits growth of at least one of *Escherichia, Salmonella* and *Listeria.* The present inventors developed an efficient screening method and surprisingly found that especially good growth inhibition can be achieved by particular combinations of lactic acid bacteria and fermentable food substrates.

According, a first aspect of the present invention is directed to a method for screening of antimicrobial effects of a ferment. This method identifies ferments that suitably inhibit growth of pathogens such as *Escherichia, Salmonella* and/or *Listeria.* As detailed herein, the method is based on determining growth inhibition of said food pathogen by qPCR and allows rapid identification of suitable ferments.

Accordingly, in further aspects, the invention is directed to a foodstuff preservative, and to a method for providing such foodstuff preservative, that inhibits growth of *Escherichia, Salmonella* and/or *Listeria* (herein also referred to as food pathogen), preferably *Salmonella enterica,* in particular *Salmonella typhimurium, Escherichia coli and*/*or Listeria monocytogenes.*

The method for providing the foodstuff preservative comprises combining lactic acid bacteria and a fermentable food substrate to obtain a ferment precursor. It further comprises incubating the ferment precursor to obtain a ferment that comprises a fermented food substrate. The ferment is for use as the foodstuff preservative.

The term "lactic acid bacteria" is well-known in the art to describe bacteria that produce lactic acid as the major metabolic end product of fermentation. Typically lactic acid bacteria are of the order *Lactobacillales.* Some examples of the genera associated with lactic acid bacteria are *Lactobacillus, Leuconostoc, Pediococcus, Lactococcus, Streptococcus, Aerococcus, Carnobacterium, Enterococcus, Oenococcus, Tetragenococcus, Vagococcus* and *Weissella.*

The term "fermentable food substrate" is herein used to describe a substrate that is suitable for human and/or animal consumption and is capable of being fermented. It may be appreciated that it can easily be tested whether a food substrate is fermentable, and such methods are known by a person skilled in the art. The fermentable food substrate is preferably plant-based or animal-based. The fermentable food substrate may comprise at least parts of herbs, spices, food crops, meat and/or fish. Due to the wider applicability of plant-based food substrate, for instance for vegan or vegetarian consumers, the fermentable food substrate preferably comprises at least part of at least parts of herbs, spices and/or food crops. Food crops include fruits, vegetables, tubers and grains such as rice and corn. The fermentable food substrate may in particular comprise at least part of herbs, spices, grains, tubers and/or vegetables. A spent fermentable food substrate may also be suitable. Spent is used to describe a foodstuff material that is the remainder of a preceding processing step, e.g. the extraction of oils or the harvesting of some other parts of the particular plant. For instance, the fermentable food substrate may comprise a spent herb. Other examples may include potato skins. As the remainder, or the spent material, may still be suitably used as a fermentable food substrate for the present invention, the present invention provides a method to reduce food spoilage. The spoilage may accordingly be reduced by the method as it provides a foodstuff preservative, but also by being able to use edible substances that may be considered waste as fermentable food substrate.

In preferred embodiments of the present invention, the food preservative can be used a seasoning or flavour enhancer of the food stuff with which it will be combined. This is particularly beneficial as it will increase the acceptance by the consumer.

Surprisingly good results were obtained for fermentable food substrates comprising at least part of lovage, garlic, sprouts, peppers, celery, spent fenugreek, oregano and/or wheat sprouts, more particularly for fermentable food substrates that comprise at least part of lovage, garlic, sprouts, peppers, celery, spent fenugreek, and/or wheat sprouts. Accordingly, these fermentable food substrates are most preferred.

It was also found that certain genera of lactic acid bacteria provide better inhibition of growth of *Escherichia, Salmonella* and/or *Listeria,* particularly of *Salmonella enterica,* in particular *Salmonella typhimurium, Escherichia coli and*/*or Listeria monocytogenes.* In this respect, the lactic acid bacteria that are preferably selected, belong to the genera from the group consisting of *Lactococccus, Lactobacillus, Carnobacterium, Pediococcus, Leuconostoc* and *Oenococcus.* It may be appreciated that the used lactic acid bacteria are preferably food-grade, indicating that the lactic acid bacteria are safe for human and/or animal consumption. For instance, the bacteria may be generally recognized as safe (GRAS) by the FDA for use in food. It is particularly preferred that the lactic acid bacteria is of the genus *Lactobacillus (L.)* and the bacteria is of the species selected from the group consisting of *L. gasseri, L. plantarum, L. pentosus* and *L. reuteri.* It may be that the particular strain of the species is also important to consider.

Particularly suitable strains of the species *L. reuteri* include LMG 9213T, which is accessible at BCCM (Belgian Coordinated Collections of Microorganisms) (Brussels, Belgium). This strain is herein also referred to as TTC 00.0254.

Additionally, particularly suitable strains of the species *L. gasseri, L. plantarum* and *L. pentosus* have been deposited with the Westerdijk Fungal Biodiversity Institute (Utrecht, The Netherlands) under numbers CBS 149435, CBS 149436, CBS 149433, CBS 149434 and CBS 149437 on 10 October 2022 by Netherlands Organisation for Applied Scientific Research TNO, Sylviusweg 71, 2333 BE Leiden, The Netherlands. These strains are herein also referred to as TTC 99.0137, TTC 03.0274, TTC 99.0119, 2015.035, 2015.034, respectively.

These particularly suitable strains may be classified in species as follows:
- *L. gasseri,* the strains deposited under number CBS 149435.
- *L. plantarum,* the strains deposited under number CBS 149433 and CBS 149436.
- *L. pentosus,* the strains deposited under number CBS 149434 and CBS 149437.

Accordingly, a further aspect of the invention is directed to a bacterial strain selected from the group consisting of: CBS 149435, CBS 149436, CBS 149433, CBS 149434 and CBS 149437, as deposited with the Westerdijk Fungal Biodiversity Institute on 10 October 2022 and combinations thereof. An even further aspect of the present invention is the use of these bacterial strains for the inhibition of growth of *Escherichia, Salmonella* and/or *Listeria,* preferably *Salmonella enterica,* in particular *Salmonella typhimurium, Escherichia coli and*/*or Listeria monocytogenes*

By combining the lactic acid bacteria and the fermentable food substrate, a ferment precursor is formed. This ferment precursor is incubated to allow for the lactic acid bacteria to start fermentation. Preferably incubation of the ferment precursor is performed at a temperature between 18-30 °C, preferably between 20-25 °C, such as at ambient temperature (approximately 21 °C). This temperature is typically kept generally constant, and the incubation thus preferably lasts, for a period of at least 24 hours, preferably at least 48 hours, more preferably for at least 72 hours.

The incubation results in a ferment comprising a fermented food substrate. It may be preferred for the fermentation to be allowed to continue after the incubation step, for instance after the ferment has been combined with a to be preserved foodstuff *(vide infra).* This would allow for further preservation and flavor development or enhancement. Accordingly, it may be that the ferment further comprises the lactic acid bacteria. Alternatively, it may be preferred that essentially all (*i.e.* more than 95%) the live cells of the lactic acid bacteria are removed from the ferment before it is used as a foodstuff preservative. The live cells may be inactivated by pasteurization or removed by any suitable means known in the art, such as bactofugation, filtration. This may be advantageous if it is not appreciated that the fermentation continues after the incubation. However, at least part of the active ingredients inhibiting growth of the food pathogens remains in the ferment, in order to remain functional as foodstuff preservative.

Depending on the final use of the foodstuff preservative, a post-processing step may also be applied. Suitable post-processing steps include drying, milling and/or grinding. Accordingly, the method to provide a foodstuff preservative may further comprise post-processing the ferment.

In order for the ferment to function as a foodstuff preservative, it is relevant to determine the antimicrobial effects of the ferment. The method for screening antimicrobial effects of a ferment, obtainable by the method described herein, on a food pathogen accordingly provides a further aspect of the present invention. The food pathogens may be *Escherichia coli, Salmonella typhimurium* and/or *Listeria monocytogenes.* The method comprises providing the ferment, such as an essentially cell-free ferment, and culturing the ferment in a culture medium. Preferably, the ferment is provided in a culture medium up to 50 vol% of the total volume (*i.e.* the volume of the ferment and the culture medium). A particularly suitable culture medium includes brain heart infusion (BHI) medium. BHI medium is a frequently used, and commercially available, growth medium for growing microorganisms. The method further comprises determining growth inhibition of the food pathogen by quantitative polymerase chain reaction (qPCR). qPCR may be used to quantify the amplification of a targeted piece of DNA during PCR and to determine the original amount of the targeted piece of DNA in a sample. For the method according to the present invention, typically genomic DNA of the one or more food pathogens were targeted. Accordingly, qPCR provides for a fast and easy way to determine the growth inhibition of different food pathogens.

Using the method for screening of antimicrobial effects, the present inventors found several particular combinations of lactic acid bacteria and fermentable food substrates that provide surprisingly good results in inhibiting growth of one or more food pathogens. The growth of one or more food pathogens may be inhibited completely and any food pathogens already present may even be killed by the foodstuff preservative according to the present invention. Accordingly, it is preferred that for the method to provide a foodstuff preservative, the combination of lactic acid bacteria and the food substrate is selected from the group indicated in Table 1 and any combination thereof.

**Table 1**

| **Lactic acid bacteria** | **Food substrate** |
|---|---|
| *Lactobacillus gasseri* | spent fenugreek |
| *Lactobacillus gasseri* | celery powder |
| *Lactobacillus gasseri* | oregano |
| *Lactobacillus pentosus* | wheat sprouts |
| *Lactobacillus pentosus* | garlic |
| *Lactobacillus pentosus* | chili pepper |
| *Lactobacillus pentosus* | lovage |
| *Lactobacillus reuteri* | wheat sprouts |
| *Lactobacillus reuteri* | garlic |
| *Lactobacillus reuteri* | oregano |
| *Lactobacillus plantarum* | wheat sprouts |
| *Lactobacillus plantarum* | lovage |
| *Lactobacillus plantarum* | garlic |
| *Lactobacillus plantarum* | chili pepper |
| *Lactobacillus plantarum* | spent fenugreek |
| *Lactobacillus plantarum* | oregano |

In particular, it is preferred that for the method to provide a foodstuff preservative, the combination of lactic acid bacteria and the food substrate is selected from the group indicated in Table 2 and any combination thereof.

**Table 2**

| **Lactic acid bacteria** | **Strain code** | **Deposition number** | **Food substrate** |
|---|---|---|---|
| *Lactobacillus gasseri* | TTC 99.0137 | CBS 149435 | spent fenugreek |
| *Lactobacillus gasseri* | TTC 99.0137 | CBS 149435 | celery powder |
| *Lactobacillus gasseri* | TTC 99.0317 | CBS 149435 | oregano |
| *Lactobacillus pentosus* | 2015.035 | CBS 149434 | wheat sprouts |
| *Lactobacillus pentosus* | 2015.035 | CBS 149434 | garlic |
| *Lactobacillus pentosus* | 2015.034 | CBS 149437 | garlic |
| *Lactobacillus pentosus* | 2015.035 | CBS 149434 | chili pepper |
| *Lactobacillus pentosus* | 2015.034 | CBS 149437 | lovage |
| *Lactobacillus reuteri* | TTC 00.0254 | LMG 9213T | wheat sprouts |
| *Lactobacillus reuteri* | TTC 00.0254 | LMG 9213T | garlic |
| *Lactobacillus reuteri* | TTC 00.0254 | LMG 9213T | oregano |
| *Lactobacillus plantarum* | TTC 03.0274 | CBS 149436 | wheat sprouts |
| *Lactobacillus plantarum* | TTC 03.0274 | CBS 149436 | lovage |
| *Lactobacillus plantarum* | TTC 99.0199 | CBS 149433 | lovage |
| *Lactobacillus plantarum* | TTC 03.0274 | CBS 149436 | garlic |
| *Lactobacillus plantarum* | TTC 99.0119 | CBS 149433 | garlic |
| *Lactobacillus plantarum* | TTC 99.0119 | CBS 149433 | chili pepper |
| *Lactobacillus plantarum* | TTC 03.0274 | CBS 149436 | spent fenugreek |
| *Lactobacillus plantarum* | TTC 03.0274 | CBS 149436 | oregano |
| *Lactobacillus plantarum* | TTC 99.0119 | CBS 149433 | oregano |

Accordingly, if the lactic acid bacteria is *Lactobacillus gasseri,* preferably of strain CBS 149435, it is preferred that the food substrate comprises at least part of spent fenugreek, celery powder and/or oregano. Good results for the inhibition of growth of one or more food pathogens were obtained for *Lactobacillus gasseri* of strain CBS 149435 and a food substrate comprising at least part of spent fenugreek and/or celery powder. More preferably the lactic acid bacteria is *Lactobacillus gasseri* of strain CBS 149435, and the food substrate comprises at least part of spent fenugreek.

In case the lactic acid bacteria is *Lactobacillus pentosus,* preferably of strain CBS 149434 and/or CBS 149437, it is preferred that the food substrate comprises at least part of wheat sprouts, garlic, chili pepper and/or lovage. Preferably, the *Lactobacillus pentosus* is of strain CBS 149434 and the food substrate comprises at least part of wheat sprouts, garlic and/or chili pepper. Good results for the inhibition of growth of one or more pathogens were obtained for *Lactobacillus pentosus* of strain CBS 149434 and a food substrate comprising at least part of wheat sprouts and/or garlic, preferably wheat sprouts. Another preferred embodiment is wherein the *Lactobacillus pentosus* is of strain CBS 149437 and the food substrate comprises at least part of garlic and/or lovage, preferably garlic.

Further, when the lactic acid bacteria is *Lactobacillus reuteri,* preferably of strain LMG 9213T, the preferred food substrate comprises at least part of wheat sprouts, garlic and/or oregano. More preferably, in case the lactic acid bacteria is *Lactobacillus reuteri,* preferably of strain LMG 9213T, the food substrate comprises at least part of wheat sprouts and/or garlic. The combination of *Lactobacillus reuteri* of strain LMG 9213T with a food substrate comprising at least part wheat sprouts leads to particularly good results.

In another aspect, the lactic acid bacteria may be *Lactobacillus plantarum,* preferably of strain CBS 149433 and/or CBS 149436, and the food substrate comprises at least part of wheat sprouts, lovage, garlic, chili pepper, spent fenugreek and/or oregano. Particularly good inhibition of growth of one or more food pathogens were obtained for *Lactobacillus plantarum* of strain CBS 149436 and a food substrate comprising at least part of wheat sprouts, lovage, garlic and/or spent fenugreek, particularly comprising at least part of wheat sprouts, lovage and/or garlic, preferably at least part of wheat sprouts and/or lovage, more preferably wheat sprouts. Additionally, if the *Lactobacillus plantarum* is of the strain CBS 149433, the food substrate preferably comprises at least part of garlic, lovage and/or chili pepper, preferably garlic and/or lovage, more preferably garlic.

The ferment obtainable by the method described herein may accordingly be used to provide a preserved foodstuff by combining the ferment with a to be preserved foodstuff to provide a preserved foodstuff. The preserved foodstuff may be stored for a prolonged period of time, such as at least 5 days, more preferably at least 10 days, such as at least 14 days before consumption. The invention is further directed to the preserved foodstuff.

It is known that some products, especially fresh foods and animal products are prone to spoilage. Accordingly, the preserved foodstuff preferably comprises a meat product, a fish product, pet food product and/or a chilled ready-to-eat product. Examples of chilled ready-to-eat products are fresh salads, sandwiches, sliced vegetables and sliced fruits.

The foodstuff preservative may thus be used as a preservative on a food product for human or animal consumption. However, the foodstuff preservative may also function as flavour enhancer or seasoning on a food product for human or animal consumption. The taste of the foodstuff preservative typically depends on the incubation time but also on the used lactic acid bacteria and the fermentable food substrate. A longer incubation time may result in more produced lactic acid, and accordingly a more acidic taste. Additionally, the foodstuff preservative may have a color, depending on the lactic acid bacteria and the fermentable food substrate. Due to the color, the foodstuff preservative may further function as colorant for the food product.

The foodstuff preservative may be added as seasoning on its own, but it may also be mixed with other herbs and/or spices to provide a seasoning mix. Such other herbs and/or spices may be commercially available, and examples include salt *(e.g.* NaCl), pepper, rosemary, basil, cinnamon, clove, dill, ginger, garlic, mint, vanilla, paprika, sage, etc. It may also be possible to combine the foodstuff preservative with commercially available seasoning mixes such as pumpkin spice mix, BBQ seasoning, Cajun seasoning, ranch season, curry seasoning and/or Italian seasoning. As spices and herbs are typically provided to customers in a dried and optionally grinded form, it may be preferred that the ferment is dried and optionally grinded. It may however also be possible that the ferment is added to a marinade or a broth. In such cases it may not be required to post-process, such as dry and/or grind, the ferment.

The foodstuff preservative may thus serve as preservative, but optionally also as seasoning and/or colorant. In any case, the foodstuff preservative is to inhibit growth of *Escherichia coli, Salmonella enterica,* preferably *Salmonella typhimurium,* and/or *Listeria monocytogenes* on the food product. Preferably, the foodstuff preservative serves as seasoning and preservative and to inhibit growth of *Escherichia coli, Salmonella enterica,* preferably *Salmonella typhimurium,* and *Listeria monocytogenes.*

The foodstuff preservative is typically added as an ingredient, indicating that it is not the main component of the foodstuff, but merely an additive. The foodstuff preservative may accordingly be considered a natural additive. For instance, the foodstuff preservative may be present in an amount of at most 30 wt%, preferably at most 10 wt%, more preferably at most 5 wt%, most preferably at most 2 wt%, such as at most 1 wt% based on the total weight of the food product and the added foodstuff preservative. The foodstuff preservative is therefore different from conventional fermented foods, such a cheese, yoghurt and sauerkraut.

For the purpose of clarity and a concise description features are described herein as part of the same or separate embodiments, however, it will be appreciated that the scope of the invention may include embodiments having combinations of all or some of the features described.

The invention may further be illustrated by the following nonlimiting examples.

### Example 1

Fermentable food substrates at 10 wt% in water were fermented with different species of lactic acid bacteria during 72 hours at ambient temperature (approximately 21 °C). Spent fenugreek was at 5 wt% in water. The ferments were processed to obtain cell-free ferments and these were tested for antimicrobial activity on *E. coli, S. typhimurium* and *L. monocytogenes,* cultured in brain heart infusion medium mixed with the cell free ferment up to 50 vol% of the total volume. Growth inhibition was determined by qPCR specifically targeting the genomic DNA of the three different pathogenic target species. The effects are compared to a non-interrupted growth control by using only brain heart infusion medium in the absence of ferment.

The results are provided in Table 3. Herein the percentages indicate the growth inhibition of the particular food pathogen. A percentage above 100% indicates that bacteria are also killed. A negative percentage indicates growth of the bacteria.

**Table 3**

| Bacteria | Strain code | Deposition number | Substrate | *S*. *typhimur ium* (%) | *E. coli* (%) | *L. monocyt ogenes* (%) |
|---|---|---|---|---|---|---|
| *L. gasseri* | TTC | CBS | Spent fenugreek | 110.5 | 122.0 | 132.3 |
| | 99.0137 | 149435 | | | | |
| *L. gasseri* | TTC | CBS | Celery powder | 70.4 | 73.7 | 106.5 |
| | 99.0137 | 149435 | | | | |
| *L. gasseri* | TTC | CBS | Oregano | -35.1 | -21.7 | 92.8 |
| | 99.0137 | 149435 | | | | |
| *L. pentosus* | 2015.035 | CBS | Wheat sprouts | 102.3 | 110.3 | 122.6 |
| | | 149434 | | | | |
| *L. pentosus* | 2015.035 | CBS | Garlic | 58.1 | 73.9 | 94.4 |
| | | 149434 | | | | |
| *L. pentosus* | 2015.034 | CBS | Garlic | 25.4 | 31.4 | 86.6 |
| | | 149437 | | | | |
| *L. pentosus* | 2015.035 | CBS | Chili pepper | 16.8 | 34.7 | 89.9 |
| | | 149434 | | | | |
| *L. pentosus* | 2015.034 | CBS | Lovage | 16.4 | 8.8 | 92.6 |
| | | 149437 | | | | |
| *L. plantarum* | TTC | CBS | Wheat sprouts | 104.3 | 131.5 | 121.3 |
| | 03.0274 | 149436 | | | | |
| *L. plantarum* | TTC | CBS | Lovage | 104.3 | 131.5 | 112.1 |
| | 03.0274 | 149436 | | | | |
| *L. plantarum* | TTC | CBS | Garlic | 104.3 | 101.8 | 98.6 |
| | 03.0274 | 149436 | | | | |
| *L. plantarum* | TTC | CBS | Garlic | 103.8 | 110.5 | 107.9 |
| | 99.0199 | 149433 | | | | |
| *L. plantarum* | TTC | CBS | Lovage | 99.0 | 116.1 | 92.3 |
| | 99.0199 | 149433 | | | | |
| *L. plantarum* | TTC | CBS | Chili pepper | 79.7 | 74.2 | 99.6 |
| | 99.0199 | 149433 | | | | |
| *L. plantarum* | TTC | CBS | Spent fenugreek | 53.9 | 63.8 | 83.3 |
| | 03.0274 | 149436 | | | | |
| *L. plantarum* | TTC | CBS | Oregano | -7.0 | 10.8 | 86.8 |
| | 99.0199 | 149433 | | | | |
| *L. plantarum* | TTC | CBS | Oregano | -7.6 | 5.9 | 81.4 |
| | 03.0274 | 149436 | | | | |
| *L. reuteri* | TTC | LMG | Wheat sprouts | 99.6 | 105.5 | 120.1 |
| | 00.0254 | 9213T | | | | |
| *L. reuteri* | TTC | LMG | Garlic | 95.5 | 83.6 | 85.5 |
| | 00.0254 | 9213T | | | | |
| *L. reuteri* | TTC | LMG | Oregano | 0.4 | 7.8 | 84.2 |
| | 00.0254 | 9213T | | | | |

## Claims

1. Method for providing a foodstuff preservative that inhibits growth of *Escherichia, Salmonella* and/or *Listeria,* preferably *Salmonella enterica,* in particular *Salmonella typhimurium, Escherichia coli and*/*or Listeria monocytogenes,* wherein the method comprises
- combining lactic acid bacteria and a fermentable food substrate to obtain a ferment precursor;
- incubating the ferment precursor to obtain a ferment that comprises a fermented food substrate, wherein said ferment is for use as the foodstuff preservative.

2. Method according to the previous claim, wherein the lactic acid bacteria is of the genus selected from the group consisting of *Lactococccus, Lactobacillus, Carnobacterium, Pediococcus, Leuconostoc* and *Oenococcus,* preferably wherein the lactic acid bacteria is of the genus *Lactobacillus* and the bacteria is of the species selected from the group consisting of *Lactobacillus gasseri, Lactobacillus plantarum, Lactobacillus pentosus* and *Lactobacillus reuteri.*

3. Method according to any of the previous claims, wherein the lactic acid bacteria is one or more selected from the group consisting of;
- *Lactobacillus gasseri* of strain CBS 149435, deposited with the Westerdijk Fungal Biodiversity Institute on 10 October 2022;
- *Lactobacillus plantarum,* of strain CBS 149436 and CBS 149433, deposited with the Westerdijk Fungal Biodiversity Institute on 10 October 2022;
- *Lactobacillus pentosus,* of strain CBS 149434 and CBS 149437, deposited with the Westerdijk Fungal Biodiversity Institute on 10 October 2022;
- *Lactobacillus reuteri,* of strain LMG 9213T, which is accessible at BCCM (Belgian Coordinated Collections of Microorganisms).

4. Method according to any of the previous claims, wherein the ferment precursor is incubated at a temperature between 18-30 °C, preferably between 20-25 °C and/or for at least 24 hours, preferably at least 48 hours, more preferably for at least 72 hours.

5. Method according to any of the previous claims, wherein the fermentable food substrate is plant-based or animal-based.

6. Method according to any of the previous claims, wherein fermentable food substrate comprises at least parts of herbs, spices, food crops, meat and/or fish, preferably wherein the fermentable food substrate comprises at least part of herbs, spices, grains, tubers and/or vegetables, more preferably wherein the fermentable food substrate comprises at least part of lovage, garlic, sprouts, peppers, celery, spent fenugreek, oregano and/or wheat sprouts, most preferably wherein the fermentable food substrate comprises at least part of lovage, garlic, sprouts, peppers, celery, spent fenugreek, and/or wheat sprouts.

7. Method according to any of the previous claims, wherein a combination of lactic acid bacteria and the food substrate is selected from the group consisting of
- *Lactobacillus gasseri* and spent fenugreek,
- *Lactobacillus gasseri* and celery powder,
- *Lactobacillus gasseri* and oregano,
- *Lactobacillus pentosus* and wheat sprouts,
- *Lactobacillus pentosus* and garlic,
- *Lactobacillus pentosus* and chili pepper,
- *Lactobacillus pentosus* and lovage,
- *Lactobacillus reuteri* and wheat sprouts,
- *Lactobacillus reuteri* and garlic,
- *Lactobacillus reuteri* and oregano,
- *Lactobacillus plantarum* and wheat sprouts,
- *Lactobacillus plantarum* and lovage,
- *Lactobacillus plantarum* and garlic,
- *Lactobacillus plantarum* and chili pepper,
- *Lactobacillus plantarum* and spent fenugreek,
- *Lactobacillus plantarum* and oregano,
and combinations thereof.

8. Method according to any of the previous claims, wherein the lactic acid bacteria is *Lactobacillus gasseri,* preferably of strain CBS 149435, and the food substrate comprises at least part of spent fenugreek, celery powder and/or oregano, preferably the lactic acid bacteria is *Lactobacillus gasseri* of strain CBS 149435 and the food substrate comprises at least part of spent fenugreek.

9. Method according to any of the previous claims 1-7, wherein the lactic acid bacteria is *Lactobacillus pentosus,* preferably of strain CBS 149434 and/or CBS 149437, and the food substrate comprises at least part of wheat sprouts, garlic, chili pepper and/or lovage, preferably wherein the lactic acid bacteria is *Lactobacillus pentosus* of strain CBS 149434 and the food substrate comprises at least part of wheat sprouts.

10. Method according to any of the previous claims 1-7, wherein the lactic acid bacteria is *Lactobacillus reuteri,* preferably of strain LMG 9213T, and the food substrate comprises at least part of wheat sprouts, garlic and/or oregano, preferably wherein the lactic acid bacteria is *Lactobacillus reuteri* of strain LMG 9213T and the food substrate comprises at least part of wheat sprouts.

11. Method according to any of the previous claims 1-7, wherein the lactic acid bacteria is *Lactobacillus plantarum,* preferably of strain CBS 149433 and/or CBS 149436, and the food substrate comprises at least part of wheat sprouts, lovage, garlic, chili pepper, spent fenugreek and/or oregano, preferably the lactic acid bacteria is *Lactobacillus plantarum* of strain CBS 149436 and the food substrate comprises at least part of wheat sprouts, lovage or garlic, more preferably at least part of wheat sprouts.

12. Foodstuff preservative obtainable by the method according to any of the previous claims.

13. Method for providing a preserved foodstuff, said method comprising providing the foodstuff preservative in a method according to any of claims 1-11, followed by combining the foodstuff preservative with a to be preserved foodstuff to obtain a preserved foodstuff.

14. Preserved foodstuff obtainable by the method according to the previous claim.

15. Method for screening of antimicrobial effects of a ferment obtainable by the method according to any of claims 1-11 on a food pathogen, preferably on *Escherichia coli, Salmonella typhimurium* and *Listeria monocytogenes,* said method for screening comprising providing the ferment, culturing said ferment in a culture medium, preferably in brain heart infusion medium, followed by determining growth inhibition of said food pathogen by qPCR.

16. Method for providing the preserved foodstuff according to claim 13 and/or the preserved foodstuff according to claim 14, wherein the preserved foodstuff comprises a meat product, a fish product, pet food product and/or a chilled ready-to-eat product.

17. Use of a foodstuff preservative in accordance with claim 12 as preservative and optional seasoning on a food product for human or animal consumption to inhibit growth of *Escherichia coli, Salmonella enterica,* preferably *Salmonella typhimurium,* and/or *Listeria monocytogenes* on said food product.

18. Use of the foodstuff preservative in accordance with claim 17, wherein said foodstuff preservative is used as seasoning and preservative to inhibit growth of *Escherichia coli, Salmonella enterica,* preferably *Salmonella typhimurium,* and *Listeria monocytogenes.*

19. *Lactobacillus* bacterial strain selected from the group consisting of: CBS 149435, CBS 149436, CBS 149433, CBS 149434 and CBS 149437, as deposited with the Westerdijk Fungal Biodiversity Institute on 10 October 2022 or any combination thereof.

20. Use of a *Lactobacillus* bacterial strain according to claim 19, as a growth inhibitor of *Escherichia, Salmonella* and/or *Listeria,* preferably *Salmonella enterica,* in particular *Salmonella typhimurium, Escherichia coli and*/*or Listeria monocytogenes.*
